# EUROPEAN PATENT SPECIFICATION

(11) **EP 1 644 044 B1**
(45) Date of publication and mention of the grant of the patent: **18.01.2012**
(21) Application number: 04743216.6
(22) Date of filing: 01.07.2004
(51) Int. Cl.: A61K 47/48

(54) **MODIFIED ANTIBODY FRAGMENTS**
MODIFIZIERTE ANTIKÖRPER-FRAGMENTE
FRAGMENTS D'ANTICORPS MODIFIES

(30) Priority: 01.07.2003 GB 0315450
(43) Date of publication of application: 12.04.2006
(73) Proprietor: UCB Pharma, S.A., 1070 Brussels (BE)
(72) Inventor: HUMPHREYS, David Paul, c/o Celltech R & D Limited, Slough, Berkshire SL1 3WE (GB); HEYWOOD, Sam Philip, c/o Celltech R & D Limited, Slough, Berkshire SL1 3WE (GB); CARRINGTON, Bruce, c/o Celltech R & D Limited, Slough, Berkshire SL1 3WE (GB)
(74) Representative: Thompson, John
(86) International application number: PCT/GB2004/002870
(87) International publication number: WO 2005/003170

(56) References cited:
- EP-A- 1 419 786
- WO-A-97/36932
- WO-A-99/15549
- HUMPHREYS D P ET AL: "Formation of dimeric Fabs in Escherichia coli: effect of hinge size and isotype, presence of interchain disulphide bond, Fab' expression levels, tail piece sequences and growth conditions" JOURNAL OF IMMUNOLOGICAL METHODS, ELSEVIER SCIENCE PUBLISHERS B.V.,AMSTERDAM, NL, vol. 209, no. 2, 1997, pages 193-202, XP004103797 ISSN: 0022-1759
- HUMPHREYS D P ET AL: "Efficient site specific removal of a C-terminal FLAG fusion from a Fab' using copper(II) ion catalysed protein cleavage" PROTEIN ENGINEERING, OXFORD UNIVERSITY PRESS, SURREY, GB, vol. 12, no. 2, February 1999 (1999-02), pages 179-184, XP000882102 ISSN: 0269-2139
- BURMEISTER GETZ E ET AL: "Comparison between the sulfhydryl reductants tris(2-carboxyethyl)phosphine and dithiothreitol for use in protein biochemistry" ANALYTICAL BIOCHEMISTRY, ACADEMIC PRESS, SAN DIEGO, CA, US, vol. 273, 15 August 1999 (1999-08-15), pages 73-80, XP000946001 ISSN: 0003-2697

## Description

The present invention relates to improved antibody fragments and more specifically provides improved antibody fragments to which one or more effector molecules are attached and methods for their production.

The high specificity and affinity of antibody variable regions make them ideal diagnostic and therapeutic agents, particularly for modulating protein:protein interactions. Antibody fragments are proving to be versatile therapeutic agents, as seen by the recent success of products such as ReoPro®. The targeting function encoded in Fv, Fab, Fab', F(ab)₂ and other antibody fragments can be used directly or can be conjugated to one or more effector molecules such as cytotoxic drugs, toxins or polymer molecules to increase efficacy. For example, since these fragments lack an Fc region they have a short circulating half-life in animals but this can be improved by conjugation to certain types of polymer such as polyethylene glycol (PEG). Increasing the size of the conjugated PEG has been shown to increase the circulating half-life from minutes to many hours and modification of a Fab' with PEG ranging from 5kDa to 100kDa has been demonstrated (Chapman et al., 1999, Nature Biotechnology, 17, 780-783; Leong et al., 2001, Cytokine, 16, 106-119; Chapman, 2002, Advanced Drug Delivery Reviews, 54, 531-545). PEGylated antibody fragments such as CDP870 are currently undergoing clinical trials where the effect of the conjugated PEG is to bring the circulating half-life to acceptable levels for therapy.

Effector molecules may be attached to antibody fragments by a number of different methods, including through aldehyde sugars or more commonly through any available amino acid side-chain or terminal amino acid functional group located in the antibody fragment, for example any free amino, imino, thiol, hydroxyl or carboxyl group. The site of attachment of effector molecules can be either random or site specific.

WO97/36932 discloses a super antigen with the ability to activate a subset of T cell, said antigen conjugated to a target seeking moiety such as an antibody.

Random attachment is often achieved through amino acids such as lysine and this results in effector molecules being attached at a number of sites throughout the antibody fragment depending on the position of the lysines. While this has been successful in some cases the exact location and number of effector molecules attached cannot be controlled and this can lead to loss of activity for example if too few are attached and/or loss of affinity if for example they interfere with the binding site (Chapman, 2002, Advanced Drug Delivery Reviews, 54, 531-545). As a result, controlled site specific attachment of effector molecules is usually the method of choice.

Site specific attachment of effector molecules is most commonly achieved by attachment to cysteine residues since such residues are relatively uncommon in antibody fragments. Antibody hinges are popular regions for site specific attachment since these contain cysteine residues and are remote from other regions of the antibody likely to be involved in antigen binding. Suitable hinges either occur naturally in the fragment or may be created using recombinant DNA techniques (See for example US 5,677,425; WO98/25971; Leong et al., 2001 Cytokine, 16, 106-119; Chapman et al., 1999 Nature Biotechnology, 17, 780-783). Alternatively, site specific cysteines may be engineered into the antibody fragment for example to create surface exposed cysteine(s) (US 5,219,996).

Humphreys D P et al J. Immunol. Meth. Vol. 209, 1997 pages 193-202 discribes the formation of dimeric Fabs in *E. coli:* effect of hinge size and isotype, presence of interchain disulphide bond, Fab' expression levels, tail piece sequences and growth conditions.

Humphreys D P et al Prot. Engineer vol. 12, 1999 pages 179-184 describes efficient site specific removal of a C-terminal FLAG fusion from a Fab' using copper (II) ion catalysed protein cleavage.

Burmeister Getz et al Analyt. Biochem. Vol. 273, 1999, pages 73-80 describes a comparison between the sulfhydryl reductants tris(2-carboxyethyl)phosphine and dithiothreitol for use in protein biochemistry.

EP 1419786 discloses chemical conjugates between an Fab fragment and molecular entities imparting diagnostic or therapeutic utility.

Where effector molecules are to be site specifically attached via a cysteine, the target thiol in the antibody fragment is often capped by a small fermentation related peptide product such as glutathione or deliberately capped by a chemical additive used during antibody fragment extraction and purification such as 5,5'-dithiobis (2-nitrobenzoic acid) (DTNB). These capping agents need to be removed to activate the target (hinge or surface) thiol. Antibody fragments have a native interchain disulphide bond between the heavy and light chain constant regions (C_{H}1 and C_{L}) that has generally been regarded as critical in maintaining the stability and binding properties of the antibody. As a result the activation of the target hinge or surface thiol must be carried out with some care such that the inter C_{L}:C_{H}1 disulphide remains intact. Hence 'mild' reducing conditions are conventionally used to remove the thiol capping agent prior to reaction with the effector molecule. This is usually achieved by using thiol based reductants such as β-mercaptoethanol (β-ME), β-mercaptoethylamine (β-MA) and dithiothreitol (DTT). However, each of these reductants is known to be able to react with and stay attached to the cysteine which it is meant to reduce (Begg and Speicher 1999 Journal of Biomolecular techniques, 10,17-20) thereby reducing the efficiency of effector molecule attachment. Hence, following reduction and reaction with effector molecules, a large proportion of the antibody fragments do not have any effector molecules attached and these have to be purified away from the antibody fragments that have the correct number of effector molecules attached. This poor efficiency of modification is clearly a disadvantage during the large-scale production of modified therapeutic antibody fragments where it is important that maximum production efficiency is achieved.

The present invention provides an antibody Fab' fragment to which at least one effector molecule is attached characterized in that the hinge contains one or two cysteines and the heavy chain in the fragment is not covalently bonded to the light chain and both the interchain cysteine of C_{L} and the interchain cysteine of C_{H}1 have been replaced with another amino acid and wherein each effector molecule is PEG and each effector molecule is attached to a cysteine in the hinge region.

Despite the absence of any covalent linkage between the heavy and the light chain and the attachment of one or more effector molecules, the fragments of the invention perform comparably with wild type fragments in a number of *in vitro* and *in vivo* tests. Suprisingly these novel fragments have the same affinity for antigen and similar *in vivo* and *in vitro* stability as wild type fragments. A particular advantage of the fragments of the invention lies in their ease of manufacture and in particular, their efficiency of manufacture. The fragments thus provide a low cost alternative to currently available fragments having inter-chain covalent linkages.

Described herein is an antibody Fab or Fab' fragment to which at least one effector molecule is attached characterized in that the heavy chain in the fragment is not covalently bonded to the light chain and both the interchain cysteine of C_{L} and the interchain cysteine of C_{H}1 have been substituted with another amino acid.

The antibody fragment described herein may comprise any heavy chain and light chain pair having a variable (V_{H}/V_{L}) and constant region (C_{H}/C_{L}). The heavy and/or light chain constant region may be extended at its C-terminal with one or more amino acids. Particular examples include Fab and Fab' fragments.

The antibody fragment starting material may be obtained from any whole antibody, especially a whole monoclonal antibody, using any suitable enzymatic cleavage and/or digestion techniques, for example by treatment with pepsin. Alternatively, or in addition the antibody starting material may be prepared by the use of recombinant DNA techniques involving the manipulation and re-expression of DNA encoding antibody variable and/or constant regions. Standard molecular biology techniques may be used to modify, add or delete amino acids or domains as desired. Any alterations to the variable or constant regions are still encompassed by the terms 'variable' and 'constant' regions as used herein.

The antibody fragment starting material may be obtained from any species including for example mouse, rat, rabbit, hamster camel, llama, goat or human. Parts of the antibody fragment may be obtained from more than one species for example the antibody fragments may be chimeric. In one example the constant regions are from one species and the variable regions from another. The antibody fragment starting material may also be modified. In one example the variable region of the antibody fragment has been created using recombinant DNA engineering techniques. Such engineered versions include those created for example from natural antibody variable regions by insertions, deletions or changes in or to the amino acid sequences of the natural antibodies. Particular examples of this type include those engineered variable region domains containing at least one CDR and optionally one or more framework amino acids from one antibody and the remainder of the variable region domain from a second antibody. The methods for creating and manufacturing these antibody fragments are well known in the art (see for example, Boss et al., US 4,816,397; Cabilly et al., US 6,331,415; Shrader et al., WO 92/02551; Ward et al., 1989, Nature, 341, 544; Orlandi et al., 1989, Proc.Natl.Acad.Sci. USA, 86, 3833; Riechmann et al., 1988, Nature, 322, 323; Bird et al, 1988, Science, 242, 423; Queen et al., US 5,585,089; Adair, WO91/09967; Mountain and Adair, 1992, Biotechnol. Genet. Eng. Rev, 10, 1-142; Verma et al., 1998, Journal of Immunological Methods, 216, 165-181).

Fab' fragments described herein are extended at the C-terminus of the heavy chain by one or more amino acids. Typically the Fab' fragments described herein possess a native or a modified hinge region. The native hinge region is the hinge region normally associated with the C_{H}1 domain of the antibody molecule. A modified hinge region is any hinge that differs in length and/or composition from the native hinge region. Such hinges can include hinge regions from any other species, such as human, mouse, rat, rabbit, hamster, camel, llama or goat hinge regions. Other modified hinge regions may comprise a complete hinge region derived from an antibody of a different class or subclass from that of the C_{H}1 domain. Thus, for instance, a C_{H}1 domain of class γ1 may be attached to a hinge region of class γ4. Alternatively, the modified hinge region may comprise part of a natural hinge or a repeating unit in which each unit in the repeat is derived from a natural hinge region. In a further alternative, the natural hinge region may be altered by converting one or more cysteine or other residues into neutral residues, such as alanine, or by converting suitably placed residues into cysteine residues. By such means the number of cysteine residues in the hinge region may be increased or decreased. In addition other characteristics of the hinge can be controlled, such as the distance of the hinge cysteine(s) from the light chain interchain cysteine, the distance between the cysteines of the hinge and the composition of other amino acids in the hinge that may affect properties of the hinge such as flexibility e.g. glycines may be incorporated into the hinge to increase rotational flexibility or prolines may be incorporated to reduce flexibility. Alternatively combinations of charged or hydrophobic residues may be incorporated into the hinge to confer multimerisation properties. Other modified hinge regions may be entirely synthetic and may be designed to possess desired properties such as length, composition and flexibility.

A number of modified hinge regions have already been described for example, in US5,677,425, WO9915549, and WO9825971. Typically hinge regions described herein will contain between 1 and 11 cysteines, Preferably between 1 and 4 cysteines and more preferably 1 or 2 cysteines. Particularly useful hinges include a modified human γ1 hinge in which only one cysteine is present, comprising the sequence DKTHTCPP (SEQ ID NO:1)or DKTHTCAA (SEQ ID NO:2) and those containing two cysteines comprising the sequence DKTHTCPPCPA (SEQ ID NO:3) or DKTHTCAACPA (SEQ ID NO:4).

The antibody fragment of the present invention will in general be capable of selectively binding to an antigen. The antigen may be any cell-associated antigen, for example a cell surface antigen on cells such as bacterial cells, yeast cells, T-cells, endothelial cells or tumour cells, or it may be a soluble antigen. Antigens may also be any medically relevant antigen such as those antigens upregulated during disease or infection, for example receptors and/or their corresponding ligands. Particular examples of cell surface antigens include adhesion molecules, for example integrins such as β1 integrins e.g. VLA-4, E-selectin, P selectin or L-selectin, CD2, CD3, CD4, CD5, CD7, CD8, CD11a, CD11b, CD18, CD19, CD20, CD23, CD25, CD33, CD38, CD40, CD45, CDW52, CD69, carcinoembryonic antigen (CEA), human milk fat globulin (HMFG1 and 2), MHC Class I and MHC Class II antigens, and VEGF, and where appropriate, receptors thereof. Soluble antigens include interleukins such as IL-1, IL-2, IL-3, IL-4, IL-5, IL-6, IL-8, IL-12, IL-16 or IL-17, viral antigens for example respiratory syncytial virus or cytomegalovirus antigens, immunoglobulins, such as IgE, interferons such as interferon α, interferon β or interferon γ, tumour necrosis factor-α, tumor necrosis factor-β, colony stimulating factors such as G-CSF or GM-CSF, and platelet derived growth factors such as PDGF-α, and PDGF-β and where appropriate receptors thereof. The term effector molecule as used herein includes, for example, antineoplastic agents, drugs, toxins (such as enzymatically active toxins of bacterial or plant origin and fragments thereof e.g. ricin and fragments thereof) biologically active proteins, for example enzymes, other antibody or antibody fragments, synthetic or naturally occurring polymers, nucleic acids and fragments thereof e.g. DNA, RNA and fragments thereof, radionuclides, particularly radioiodide, radioisotopes, chelated metals, nanoparticles and reporter groups such as fluorescent compounds or compounds which may be detected by NMR or ESR spectroscopy.

Particular antineoplastic agents include cytotoxic and cytostatic agents for example alkylating agents, such as nitrogen mustards (e.g. chlorambucil, melphalan, mechlorethamine, cyclosphophamide, or uracil mustard) and derivatives thereof, triethylenephosphoramide , triethylenethiophosphor-amide, busulphan, or cisplatin; antimetabolites, such as methotrexate, fluorouracil, floxuridine, cytarabine, mercaptopurine, thioguanine, fluoroacetic acid, or fluorocitric acid, antibiotics, such as bleomycins (e.g. bleomycin sulphate), doxorubicin, daunorubicin, mitomycins (e.g. mitomycin C), actionmycins (e.g. dactinomycin) plicamyin, calichaemicin and derivatives thereof, or esperamicin and derivatives thereof; mitotic inhibitors, such as etoposide, vincristine or vinblastine and derivatives thereof; alkaloids such as ellipticine; polyols such as taxicin-I or taxicin-II; hormones, such as androgens (e.g. dromostanolone or testolactone), progestins (e.g. megestrol acetate or medroxyprogesterone acetate), estrogens (e.g. dimethylstilbestrol diphosphate, polyestradiol phosphate or estramustine phosphate) or antiestrogens (e.g. tamoxifen); anthraquinones, such as mitoxantrone, ureas, such as hydroxyurea; hydrazines, such as procarbazine; or imidazoles, such as dacarbazine.

Chelated metals include chelates of di- or tripositive metals having a coordination number from 2 to 8 inclusive. Particular examples of such metals include technetium (Tc), rhenium (Re), cobalt (Co), copper (Cu), gold (Au), silver (Ag), lead (Pb), bismuth (Bi), indium (In), gallium (Ga), yttrium (Y), terbium (Tb), gadolinium (Gd), and scandium (Sc). In general the metal is preferably a radionuclide. Particular radionuclides include ^{99m}Tc, ¹⁸⁶Re, ¹⁸⁸Re, ⁵⁸Co, ⁶⁰Co ⁶⁷Cu, ¹⁹⁵Au, ¹⁹⁹Au, ¹¹⁰Ag, ²⁰³Pb, ²⁰⁶Bi, ²⁰⁷Bi, ¹¹¹In, ⁶⁷Ga, ⁶⁸Ga, ⁸⁸Y, ⁹⁰Y, ¹⁶⁰Tb, ¹⁵³Gd and ⁴⁷Sc.

The chelated metal may be for example one of the above types of metal chelated with any suitable polyadentate chelating agent, for example acyclic or cyclic polyamines, polyethers, (e.g. crown ethers and derivatives thereof); polyamides; porphyrins; and carbocyclic derivatives.

In general, the type of chelating agent will depend on the metal in use. One particularly useful group of chelating agents in conjugates according to the invention, however, are acyclic and cyclic polyamines, especially polyaminocarboxylic acids, for example diethylenetriaminepentaacetic acid and derivatives thereof, and macrocyclic amines, e.g. cyclic tri-aza and tetra-aza derivatives (for example as described in International Patent Specification No. WO 92/22583); and polyamides, especially desferriox-amine and derivatives thereof.

Other effector molecules include proteins, peptides and enzymes. Enzymes of interest include, but are not limited to, proteolytic enzymes, hydrolases, lyases, isomerases, transferases. Proteins, polypeptides and peptides of interest include, but are not limited to, immunoglobulins, toxins such as abrin, ricin A, pseudomonas exotoxin, or diphtheria toxin, a protein such as insulin, tumour necrosis factor, α-interferon, β-interferon, nerve growth factor, platelet derived growth factor or tissue plasminogen activator, a thrombotic agent or an anti-angiogenic agent, e.g. angiostatin or endostatin, or, a biological response modifier such as a lymphokine, interleukin-1 (IL-1), interleukin-2 (IL-2), interleukin-6 (IL-6), granulocyte macrophage colony stimulating factor (GM-CSF), granulocyte colony stimulating factor (G-CSF), nerve growth factor (NGF) or other growth factor and immunoglobulins.

Other effector molecules may include detectable substances useful for example in diagnosis. Examples of detectable substances include various enzymes, prosthetic groups, fluorescent materials, luminescent materials, bioluminescent materials, radioactive nuclides, positron emitting metals (for use in positron emission tomography), and nonradioactive paramagnetic metal ions. See generally U.S. Patent No. 4,741,900 for metal ions which can be conjugated to antibodies for use as diagnostics. Suitable enzymes include horseradish peroxidase, alkaline phosphatase, beta-galactosidase, or acetylcholinesterase; suitable prosthetic groups include streptavidin, avidin and biotin; suitable fluorescent materials include umbelliferone, fluorescein, fluorescein isothiocyanate, rhodamine, dichlorotriazinylamine fluorescein, dansyl chloride and phycoerythrin; suitable luminescent materials include luminol; suitable bioluminescent materials include luciferase, luciferin, and aequorin; and suitable radioactive nuclides include ¹²⁵I, ¹³¹I, ¹¹¹In and ⁹⁹Tc.

Synthetic or naturally occurring polymers for use as effector molecules include, for example optionally substituted straight or branched chain polyalkylene, polyalkenylene, or polyoxyalkylene polymers or branched or unbranched polysaccharides, e.g. a homo- or hetero- polysaccharide such as lactose, amylose, dextran or glycogen.

Particular optional substituents which may be present on the above-mentioned synthetic polymers include one or more hydroxy, methyl or methoxy groups. Particular examples of synthetic polymers include optionally substituted straight or branched chain poly(ethyleneglycol), poly(propyleneglycol), poly(vinylalcohol) or derivatives thereof, especially optionally substituted poly(ethyleneglycol) such as methoxypoly(ethyleneglycol) or derivatives thereof.

"Derivatives" as used herein is intended to include reactive derivatives, for example thiol-selective reactive groups such as an α-halocaraboxylic acid or ester, e.g. iodoacetamide, an imide, e.g. maleimide, a vinyl sulphone or disulphide malemides and the like. The reactive group may be linked directly or through a linker segment to the polymer. It will be appreciated that the residue of such a group will in some instances form part of the product as the linking group between the antibody fragment and the polymer.

The size of each polymer molecule may be varied as desired, but will generally be in an average molecular weight range from 500Da to 50,000Da, preferably from 5,000 to 40,000Da and more preferably from 10,000 to 40,000Da and 20,000 to 40,000Da. The polymer size may in particular be selected on the basis of the intended use of the product for example ability to localize to certain tissues such as tumors or extend circulating half life (for review see Chapman, 2002, Advanced Drug Delivery Reviews, 54, 531 -545). Thus, for example, where the product is intended to leave the circulation and penetrate tissue, for example for use in the treatment of a tumor, it may be advantageous to use a small molecular weight polymer, for example with a molecular weight of around 5,000Da. For applications where the product remains in the circulation, it may be advantageous to use a higher molecular weight polymer, for example having a molecular weight in the range from 25,000Da to 40,000Da.

Particularly preferred polymers include a polyalkylene polymer, such as a poly(ethyleneglycol) or, especially, a methoxypoly(ethyleneglycol) or a derivative thereof, and especially with a molecular weight in the range from about 10,000Da to about 40,000Da.

The polymers described herein may be obtained commercially (for example from Nippon Oil and Fats;Nektar Therapeutics) or may be prepared from commercially available starting materials using conventional chemical procedures.

Effector molecules described herein may be attached using standard chemical or recombinant DNA procedures in which the protein is linked either directly or via a coupling agent to the effector molecule. Techniques for conjugating such effector molecules to antibodies are well known in the art (see, Hellstrom et al., Controlled Drug Delivery, 2nd Ed., Robinson et al., eds., 1987, pp. 623-53; Thorpe et al., 1982 , Immunol. Rev., 62:119-58 and Dubowchik et al., 1999, Pharmacology and Therapeutics, 83, 67-123). Particular chemical procedures include for example those described in International Patent Specification numbers WO 93/06231, WO92/22583, WO90/09195, WO89/01476, WO99155 and WO03031581. Alternatively, where the effector molecule is a protein or polypeptide the linkage may be achieved using recombinant DNA procedures, for example as described in European Patent Specification No. 392745.

In one example the effector molecules described herein may be attached to the protein through any available amino acid side-chain or terminal amino acid functional group located in the antibody fragment, for example any free amino, imino, thiol, hydroxyl or carboxyl group. Such amino acids may occur naturally in the antibody fragment or may be engineered into the fragment using recombinant DNA methods. See for example US 5,219,996. An effector molecule may be covalently linked through a thiol group of a cysteine residue located in the fragment. The covalent linkage will generally be a disulphide bond or, in particular, a sulphur-carbon bond. In one example where a thiol group is used as the point of attachment appropriately activated effector molecules, for example thiol selective derivatives such as maleimide and cysteine derivatives may be used.

It will be appreciated that where there are two or more effector molecules attached to the antibody fragment these may be identical or different and may be attached to the antibody fragment at different sites. It will also be appreciated that two or more effector molecules may be attached to the antibody fragment at a single site by the use for example of a branched connecting structure to link two or more effector molecules and provide a single site of attachment.

In the present invention the effector molecules attached to the antibody fragment is a PEG molecule, . As regards attaching poly(ethyleneglycol) (PEG) moieties in general, reference is made to "Poly(ethyleneglycol) Chemistry, Biotechnical and Biomedical Applications", 1992, J.Milton Harris (ed), Plenum Press, New York; "Poly(ethyleneglycol) Chemistry and Biological Applications", 1997, J. Milton Harris and S.Zalipsky (eds), American Chemical Society , Washington DC and "Bioconjugation Protein Coupling Techniques for the Biomedical Sciences", 1998, M. Aslam and A. Dent, Grove Publishers, New York.

In one example of the present invention all the effector molecules attached to the fragment are PEG and each molecule is covalently linked via a maleimide group to one or more thiol groups in the antibody fragment. The PEG may be any straight or branched molecule. To attach branched PEG molecules, a lysine residue is preferably covalently linked to the maleimide group. To each of the amine groups on the lysine residue is preferably attached a methoxy(poly(ethyleneglycol) polymer. In one example the molecular weight of each polymer is approximately 20,000Da and the total molecular weight of the entire polymer molecule is therefore approximately 40,000Da.

The term interchain cysteine as used herein refers to a cysteine in the heavy or light chain constant region that would be disulphide linked to a cysteine in the corresponding heavy or light chain constant region in a naturally occurring antibody molecule. In particular the interchain cysteines of the present invention are a cysteine in the constant region of the light chain (C_{L}) and a cysteine in the first constant region of the heavy chain (C_{H}1) that are disulphide linked to each other in naturally occurring antibodies. Examples of such cysteines may typically be found at position 214 of the light chain and 233 of the heavy chain of human IgG1, 127 of the heavy chain of human IgM, IgE, IgG2, IgG3, IgG4 and 128 of the heavy chain of human IgD and IgA2B, as defined by Kabat et al., 1987, in Sequences of Proteins of Immunological Interest, US Department of Health and Human Services, NIH, USA. It will be appreciated that the exact positions of these cysteines may vary from that of naturally occurring antibodies if any modifications, such as deletions, insertions and/or substitutions have been made to the antibody starting material.

In the modified fragments of the present invention the disulphide linkage between the interchain cysteine of C_{L} and the interchain cysteine of C_{H}1 is absent. As a result the heavy chain in the antibody fragment is not covalently bonded to the light chain. In the present invention the interchain cysteines in the antibody fragment starting material have both been replaced by another amino acid, preferably an amino acid that does not contain a thiol group. By replace we mean that where the interchain cysteine would normally be found in the antibody fragment another amino acid is in its place. Examples of suitable amino acids include serine, threonine, alanine, glycine or any polar amino acid. A particularly preferred amino acid is serine. The amino acids used to replace the interchain cysteines may be the same in both chains or different from each other. The methods for replacing amino acids are well known in the art of molecular biology. Such methods include for example site directed mutagenesis using methods such as PCR to delete and/or substitute amino acids or *de novo* design of synthetic sequences. Fab' and F(ab')₂ in which both the interchain cysteines have been replaced by serines have already been described (Humphreys et al., 1997, Journal of Immunological Methods, 209, 193-202; Rodrigues et al., 1993, The Journal of Immunology, 151, 6954-6961).

In the present invention at least one effector molecule is attached to the antibody fragment. The effector molecules may be attached by any of the means described herein.

In the present invention each effector molecule is attached to a cysteine residue in the antibody fragment constant region. Additional effector molecules may be attached elsewhere in the antibody fragment, in particular the constant region and/or the hinge region using the methods described herein. Suitable cysteines for attachment include naturally occurring cysteines and cysteines that have been engineered into the fragment using recombinant DNA techniques. In one example two cysteines are engineered into the antibody fragment, one in each of the heavy and light chain constant regions. In one particular example these cysteines are engineered at positions whereby they can form a disulphide linkage with each other in the antibody starting material.

Particular fragments described herein are those where:
(i) at least one effector molecule is attached to the heavy or light chain constant region or
(ii) an effector molecule is attached to a cysteine in the light chain constant region and the heavy chain constant region or
(iii) the cysteine residues in the heavy and light chain constant regions which are attached to effector molecules would otherwise be linked to each other via a disulphide bond if the effector molecules were not attached
In another example described herein the antibody fragment is a Fab' fragment and at least one effector molecule is attached to the fragment via the hinge region, preferably via a cysteine present in the hinge region. Additional effector molecules may be attached elsewhere in the antibody fragment, in particular the constant region and/or the hinge region using the methods described wherein.

Particular fragments described herein are those where:
(i) one effector molecule is attached to the hinge region or
(ii) two effector molecules are attached to the hinge region or
(iii) all effector molecules attached to the fragment are attached to the hinge region
Also described herein are methods for attaching one or more effector molecules to the antibody Fab or Fab' fragments described herein said method comprising:
a) Treating an antibody Fab or Fab' fragment in which both the interchain cysteine of C_{L} and the interchain cysteine of C_{H}1 have been replaced with another amino acid with a reducing agent capable of generating at least one free thiol group in the fragment
b) Reacting the treated fragment with an effector molecule
The methods described herein enable one or more effector molecule(s) to be attached to cysteines in the antibody fragment, in particular to cysteines in the constant region and/or the hinge. Two or more effector molecules can be attached to the antibody fragment either simultaneously or sequentially by repeating the method.

The methods described herein also extend to one or more steps before and/or after the reduction method described above in which further effector molecules are attached to the antibody fragment using the methods described previously, for example via other available amino acids side chains such as amino or imino groups.

The reducing agent for use in the methods described herein is any reducing agent capable of reducing cysteines in the antibody fragment starting material to produce free thiols. Preferably the reducing agent efficiently reduces all available thiols. The reducing agent will need to be strong enough to reduce any disulphide bonds between the cysteines of the heavy and light chain constant regions, for example cysteines that have been engineered into the constant region, in order to allow attachment of effector molecules to said cysteines. Where there are no interchain disulphide bonds, for example where there are no engineered pairs of cysteines in the constant regions and the interchain cysteines of C_{L} and C_{H}1 have been substituted with another amino acid, the reducing agent must be capable of efficiently liberating free thiols from the remaining cysteine(s) in the antibody fragment which are not in disulphide linkage, e.g. a cysteine in the hinge region. As the antibody molecules of the present invention have no requirement for the interchain disulphide bond stronger reducing agents can be used than are conventionally used with wild type antibody fragments. As a result a higher number of free thiols are produced and a higher proportion of the antibody fragments are correctly modified i.e. the correct number of effector molecules are attached. The antibody fragments described herein can therefore be produced more efficiently and cost effectively than conventional antibody fragments. It will be clear to a person skilled in the art that suitable reducing agents may be identified by determining the number of free thiols produced after the antibody fragment is treated with the reducing agent. Methods for determining the number of free thiols are well known in the art, see for example Lyons *et al.,* 1990, Protein Engineering, 3, 703. Reducing agents for use in the methods described herein are widely known in the art for example those described in Singh et al., 1995, Methods in Enzymology, 251, 167-73. Particular examples include thiol based reducing agents such as reduced glutathione (GSH), β-mercaptoethanol (β-ME), β-mercaptoethylamine (β-MA) and dithiothreitol (DTT). Alternatively the antibody fragments described herein may be reduced using electrolytic methods, such as the method described in Leach et al., 1965, Div. Protein. Chem, 4, 23-27 and using photoreduction methods, such as the method described in Ellison et al., 2000, Biotechniques, 28 (2), 324-326. Preferably however, the reducing agent is a non-thiol based reducing agent capable of liberating one or more thiols in an antibody fragment. Preferably the non-thiol based reducing agent is capable of liberating all available thiols in an antibody fragment. The reducing agents for use in the present invention are trialkylphosphine reducing agents (Ruegg UT and Rudinger, J., 1977, Methods in Enzymology, 47, 111-126; Bums J et al., 1991, J.Org.Chem, 56, 2648-2650; Getz et al., 1999, Analytical Biochemistry, 273, 73-80; Han and Han, 1994, Analytical Biochemistry, 220, 5-10; Seitz et al., 1999, Euro.J.Nuclear Medicine, 26, 1265-1273). Particular examples of which include tris(2-carboxyethyl)phosphine (TCEP), tris butyl phosphine (TBP), tris-(2-cyanoethyl) phosphine, tris-(3-hydroxypropyl) phosphine (THP) and tris-(2-hydroxyethyl) phosphine. Most preferably the reducing agent for use in the present invention is either TCEP or THP. It will be clear to a person skilled in the art that the concentration of reducing agent for use in the present invention can be determined empirically for example, by varying the concentration of reducing agent and measuring the number of free thiols produced. Typically the reducing agent for use in the present invention is used in excess over the antibody fragment for example between 2 and 1000 fold molar excess. Preferably the reducing agent is in 2, 3, 4, 5, 10, 100 or 1000 fold excess. In one preferred example the reducing agent is in 4 molar excess.

The modified antibody fragments described herein may be prepared by reacting an antibody fragment (as described herein) containing at least one reactive cysteine residue with an effector molecule, preferably a thiol-selective activated effector molecule. The reactions in steps (a) and (b) of the method described above may generally be performed in a solvent, for example an aqueous buffer solution such as acetate or phosphate, at around neutral pH, for example around pH 4.5 to around pH 8.0. The reaction may generally be performed at any suitable temperature, for example between about 5°C and about 70°C, for example at room temperature. The solvent may optionally contain a chelating agent such as EDTA, EGTA, CDTA or DTPA. Preferably the solvent contains EDTA at between 1 and 5mM, preferably 2mM. Alternatively or in addition the solvent may be a chelating buffer such as citric acid, oxalic acid, folic acid, bicine, tricine, tris or ADA. The effector molecule will generally be employed in excess concentration relative to the concentration of the antibody fragment. Typically the effector molecule is in between 2 and 100 fold molar excess, preferably 5, 10 or 50 fold excess.

Where necessary, the desired product containing the desired number of effector molecules may be separated from any other product generated during the production process and containing an unwanted number of effector molecules by conventional means, for example by chromatography techniques such as ion exchange, size exclusion or hydrophobic interaction chromatography.

Also described herein is a mixture containing two or more antibody Fab or Fab' fragments, characterized in that the mixture is enriched for Fab or Fab' fragments in which the light chain in said fragments is not covalently bonded to the heavy chain, both the interchain cysteines of C_{L} and C_{H} have been replaced by another amino acid and at least one effector molecule is attached to the fragment. Said mixture may be produced using the methods described herein. By 'enriched' we mean that the antibody fragment with the desired number of effector molecules attached accounts for 50% or greater of the mixture. Preferably the antibody fragment with the desired number of effector molecules attached accounts for between 50 and 99% of the mixture. Preferably the mixtures are enriched by greater than 50%, preferably greater than 60%, more preferably greater than 70%. The proportion of such mixtures containing the antibody fragment with the desired number of effector molecules may be determined by using the size exclusion HPLC methods described herein. In one example the mixture is enriched with a Fab' fragment in which the light chain in said fragment is not covalently bonded to the heavy chain, both the interchain cysteines of C_{L} and C_{H}1 have been replaced by another amino acid and one effector molecule is attached to the hinge region. In another example the mixture is enriched with a Fab' fragment in which the light chain in said fragment is not covalently bonded to the heavy chain, both the interchain cysteines of C_{L} and C_{H}1 have been replaced by another amino acid and two effector molecules are attached to the hinge region.

Also described herein is a process which can be used to facilitate the isolation of soluble, correctly folded antibody fragments in which the light chain in said fragments is not covalently bonded to the heavy chain and both the interchain cysteines of C_{L} and C_{H}1 have been replaced by another amino acid. This aspect relates to the recombinant production of said antibody fragments in a host cell and is particularly suited to antibody Fab or Fab' fragments which are expressed in bacterial cells transformed with a vector comprising DNA coding for said antibodies. High temperature heat extractions are commonly used in the extraction of conventional antibodies expressed in bacterial cells (see the methods described in US 5,665,866) in order to obtain correctly folded and assembled antibody fragments, free of host proteins and antibody-related material such as free heavy and light chains or fragments thereof. Conventional antibody fragments containing an interchain disulphide linkage between the heavy and light chain constant regions are typically extracted at around 60 to 65°C. However, in one example of the present invention the Fab or Fab' fragment starting material does not have a covalent linkage between the heavy and light chains. Surprisingly we have been able to demonstrate that a heat extraction can still be used to obtain these antibody fragments despite the absence of the disulphide linkage. Hence the present invention also provides a process to facilitate the isolation of antibody Fab or Fab' fragments produced in bacterial cells in which the light chain in said fragments is not covalently bonded to the heavy chain and both the interchain cysteines of C_{L} and C_{H}1 have been replaced by another amino acid, comprising subjecting a preparation comprising said antibody Fab or Fab' fragments to an elevated temperature within the range of 34 to 59°C and recovering said antibody Fab or Fab' fragments from said preparation. Preferably the temperature for use in the process is within the range 45 to 59°C, more preferably within the range 50 to 56°C. Preferred temperatures for use in the process are 50, 51, 52, 53, 54, 55 and 56°C.

The antibody fragments according to the invention may be useful in the detection or treatment of a number of diseases or disorders. Such diseases or disorders may include those described under the general heading of infectious disease, e.g. bacterial infection, fungal infection, inflammatory disease/autoimmunity e.g. rheumatoid arthritis, osteoarthritis, inflammatory bowel disease; cancer; allergic/atopic disease e.g. asthma, eczema; congenital disease, e.g. cystic fibrosis, sickle cell anemia; dermatologic disease e.g. psoriasis; neurologic disease, e.g. multiple sclerosis; transplants e.g. organ transplant rejection, graft-versus-host disease; and metabolic/idiopathic disease e.g. diabetes.

The antibody fragments according to the invention may be formulated for use in therapy and/or diagnosis and according to a further aspect of the invention we provide a pharmaceutical composition comprising an antibody Fab or Fab' fragment to which at least one effector molecule is attached characterized in that the heavy chain in the fragment is not covalently bonded to the light chain and both the interchain cysteine of C_{L} and the interchain cysteine of C_{H}1 have been substituted with another amino acid, together with one or more pharmaceutically acceptable excipients, diluents or carriers.

### EXAMPLES

The present invention will now be described by way of example only, in which reference is made to:
Figure 1: Proportions of mono-PEGylated vs. unPEGylated g165Fab' LC-S HC-S, hinge-CAA produced using various reductants, as determined by size exclusion HPLC.
Figure 2a: Non-reducing SDS-PAGE of purified g165 Fab' variants. Lane 2 shows the purified Fab' fragment g165 Fab' LC-S HC-S, hinge CAA. Lane 6 shows the purified control Fab' fragment g165 Fab' LC-S HC-S, hinge SAA.
Figure 2b: Non-reducing SDS-PAGE of PEGylated g165 Fab' variants. Lane 2 shows the pegylated Fab' fragment g165 Fab' LC-S HC-S, hinge CAA. Lane 6 shows the control Fab' fragment g165 Fab' LC-S HC-S, hinge SAA.
Figure 3: Non-reducing SDS-PAGE of purified g165 Fab' LC-C HC-C, hinge-CAA (lanes 1-7) and g165Fab' LC-S HC-S, hinge-CAA (lanes 8-14) following overnight incubation at various temperatures in an *E.coli* periplasmic extract. Temperatures were RT, 37°C, 45°C, 45°C, 50°C, 55°C, 60°C and 65°C in lanes 1-7 and 8-14.
Figure 4: Non-reducing SDS-PAGE showing the cation exchange recovery of g165 Fab' LC-S HC-S hinge CAA following overnight periplasmic extraction in Tris/EDTA (T.E) at a range of temperatures. Lane 1 shows crude T.E. extract; Lane 2 shows crude T.E. extract at pH4.5; Lanes 3-8 show the flow through from a cation exchange column of extracts made at 30°C, 37°C, 47°C, 52°C, 56°C and 60°C respectively; lanes 9-14 show the eluates from a cation exchange column of extracts made at 30°C, 37°C, 47°C, 52°C, 56°C and 60°C respectively.
Figure 5: Protein G recovery of g165 Fab' LC-S HC-S hinge-CAA following overnight periplasmic extraction in Tris/EDTA at a range of temperatures
Figure 6: Protein G recovery of g165 Fab' LC-S HC-S hinge CAA following successive 1 hour incubations at increasing temperatures in a Tris/EDTA periplasmic extraction.
Figure 7: Pharmacokinetics of intravenously dosed ¹²⁵I labelled mono-PEGylated g8516 Fab' in rats
Figure 8: Pharmacokinetcs of subcutaneously dosed ¹²⁵I labelled mono-PEGylated g8516 Fab' in rats
Figure 9: Pharmacokinetics of intravenously dosed ¹²⁵I labelled di-PEGylated g8516 Fab' in rats
Figure 10: Neutralisation of intravenously dosed antigen-induced IL-6 generation by intravenously dosed Fab'-PEG in mice
Figure 11: Neutralisation of intraperitoneal dosed antigen-induced neutrophil accumulation by intravenous pre-dosing of g8516 Fab'-PEG in mice.
Figure 12: Non-reducing SDS-PAGE of mono and di-PEGylated g8516 Fab'. Lane 1, g8516 Fab LC-C HC-C, hinge-CAA-1x40kDa PEG; lane 2, g 8516 Fab LC-C HC-C, hinge-CPPCPA-2x20kDa PEG; lane 3, g8516 Fab LC-S HC-S, hinge-CAA-1x40kDa PEG; lane 4 g8516 Fab LC-S HC-S, hinge CPPCPA-2x20kDa PEG.

### Fab' Nomenclature and General Methods

The Fab' molecules used in the following examples are g165 Fab' which binds to a human cell surface receptor and g8516 which binds to the human cytokine IL-1β. The nomenclature for each fragment uses the single letter code C for cysteine and S for serine to denote the amino acid at the site of the inter-chain cysteine of C_{L} in the light chain (LC) and the site of the inter-chain cysteine of C_{H}1 in the heavy chain (HC). For example, a normal Fab' is 'g165 Fab' LC-C HC-C, hinge-CAA' whereas the version in which both the interchain cysteines have been substituted with a serine so there is no inter chain disulphide between C_{L} and C_{H}1 is `g165 Fab' LC-S HC-S, hinge-CAA'. A similar Fab' with a full γ1 middle hinge is noted as 'g165 Fab' LC-S HC-S, hinge-CPPCPA'. A list of the plasmids used in the following examples are shown in Table 1.

**Table 1. Plasmid and protein details.**

| **Plasmid** | **Protein** | **Disulphide structure** |
|---|---|---|
| PDPH147 | g165 Fab' LC-C, HC-C, hinge-CAA | LC-GE[?] HC-KS[?]DKTHT[?]AA |
| PDPH 197 | g165 Fab' LC-S, HC-S, hinge-CAA | LC-GES HC-KSSDKTHT[?]AA 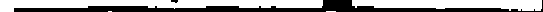 |
| PDPH238 | g8516 Fab' LC-C, HC-C, hinge CPPCPA | LC-GE[?] HC-KS[?]DKTHT[?]PP[?]PA |
| PDPH241 | g8516 Fab' LC-S, HC-S, hinge-CAA | LC-GES HC-KSSDKTHT[?]AA 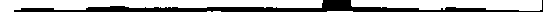 |
| PDPH242 | g8516 Fab' LC-S, HC-S, hinge-CPPCPA | LC-GES HC-KTSDKTHT[?]PP[?]PA |
| PDPH243 | g8516 Fab' LC-S, HC-S, hinge-CPSCPA | LC-GES HC-KTSDKTHT[?]PS[?]PA |

### Production of Fab'

PCR mutagenesis was used to change the interchain cysteines of C_{L} and C_{H}1 in the Fab' fragments to serines. Fab' molecules were produced in *E.coli* strain W3110 and purified using standard methods (Humphreys et al., 2002, Protein Expression and Purification, 26, 309-320).

### Reduction and PEGylation of Fab' fragments.

All reductions and PEGylations were performed in 0.1M Phosphate pH6.0; 2mM EDTA. The concentration of Fab' and reductant were as stated in each example. In all cases reduction was done for 30 minutes at room temperature (∼24°C), the proteins desalted on a PD-10 column (Pharmacia) and then mixed with 5 fold molar excess of PEG-maleimide over Fab'. The 40kDa PEG was from Nektar and 20 and 30kDa PEG was from Nippon Oil and Fats. PEGylated Fab' was separated from unpegylated Fab' by size exclusion HPLC on analytical Zorbax GF-450 and GF-250 columns in series. These were developed with a 30min isocratic gradient of 0.2M phosphate pH 7.0 + 10% ethanol at 1ml/min and Fab' detected using absorbance at 214nm and 280nm.

### Example 1: Creation of novel PEGylated Fab' fragments

The antibody Fab' fragments g165Fab' LC-S HC-S, hinge CAA and g8516 Fab' LC-S HC-S, hinge CAA were produced by replacing the interchain cysteines of C_{L} and C_{H}1 with serine by PCR mutagenesis. A single PEG fragment was attached to the hinge of each fragment by reducing the hinge cysteine and attaching PEG-maleimide to said cysteine. In the case of g165Fab' LC-S HC-S, hinge CAA a number of different reducing agents were tested at a 4 molar excess over the Fab' and these were the thiol based reductants reduced glutathione (GSH), β-mercaptoethanol (β-ME), β-mercaptoethylamine (β-MA) and dithiothreitol (DTT) and the non-thiol based reductant tris carboxyethyl phosphine (TCEP). Following reduction and PEGylation the number of PEG molecules attached to the Fab' fragments was determined by size exclusion chromatography.

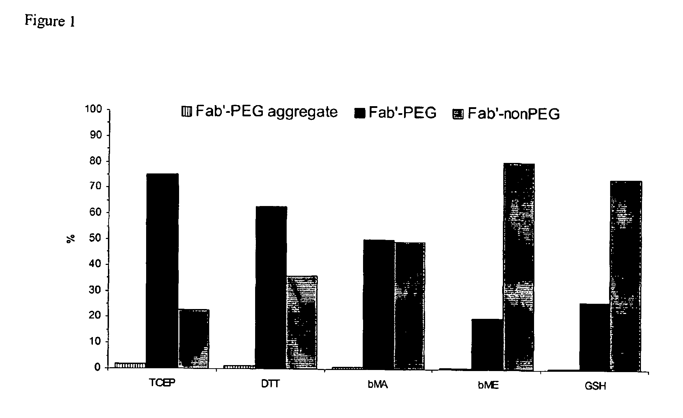

Very high levels of mono-PEGylation were obtained when TCEP was used as the reductant and the ratio of PEGylated to unPEGylated material was approximately 75:25 (Figure 1). Under these conditions the ratios for the thiol based reductants were as follows, DTT 64:36, β-MA 50:50, β-MA 20:80 and GSH 25:75. TCEP was clearly the most efficient reducing agent tested, producing a 10% increase in PEGylated material over DTT. The lack of an interchain disulphide between C_{L} and C_{H}1 enables stronger reducing agents to be used that would ordinarily reduce the interchain disulphide in a conventional Fab' fragment. In our hands PEGylation of the same antibody fragment but with the inter-chain disulphide intact (g165 Fab' LC-C HC-C, hinge-CAA), using the thiol based reductants typically resulted in a low efficiency of monoPEGylation, 55% for DTT, 52%βMA, 20%βME and 22% GSH (See also our co-pending application, GB0315457.2). The novel fragments of the present invention can therefore be produced more efficiently than conventional antibody fragments. TCEP is one example of a useful reducing agent for producing the antibody fragments of the present invention.

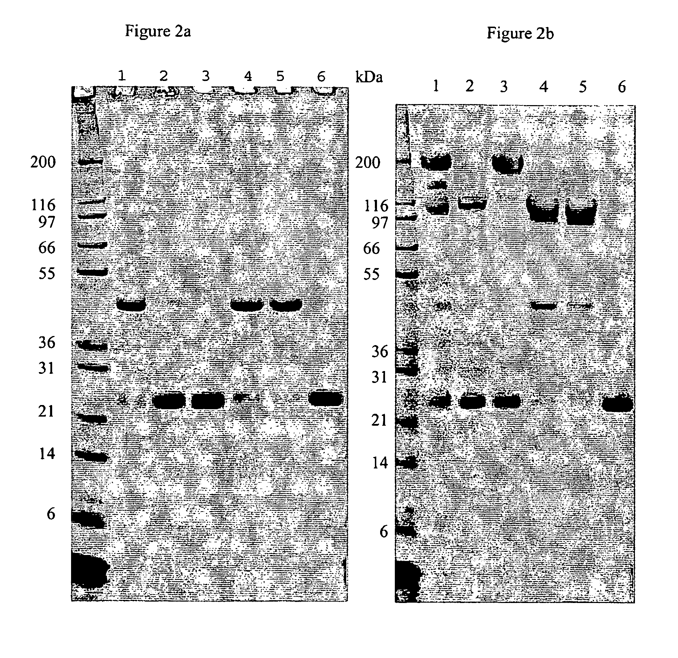

Figures 2a and 2b show that a single PEG molecule was attached to the fragment. Figure 2a shows the purified Fab' fragment g165 Fab' LC-S HC-S, hinge CAA where the single band in lane 2 represents both free heavy and light chain. Figure 2b shows the increase in mass of the heavy chain of g165 Fab' LC-S HC-S, hinge CAA (lane 2) following attachment of a single PEG molecule to the hinge (the highest molecular weight band in lane 2). The lower molecular weight band in lane 2 is free light chain.
TCEP was also used to efficiently diPEGylate the hinge of g165Fab' LC-S HC-S, hinge CPPCPA, g8516 Fab' LC-S HC-S, hinge CPPCPA and g8516 Fab' LC-S HC-S, hinge CPSCPA.
The following examples demonstrate that these new fragments have similar properties to conventional fragments that contain an interchain disulphide bond.

### Example 2 Stability tests of Fab' lacking the inter C_{L}:C_{H}1 disulphide

### Effect of temperature on purified Fab'

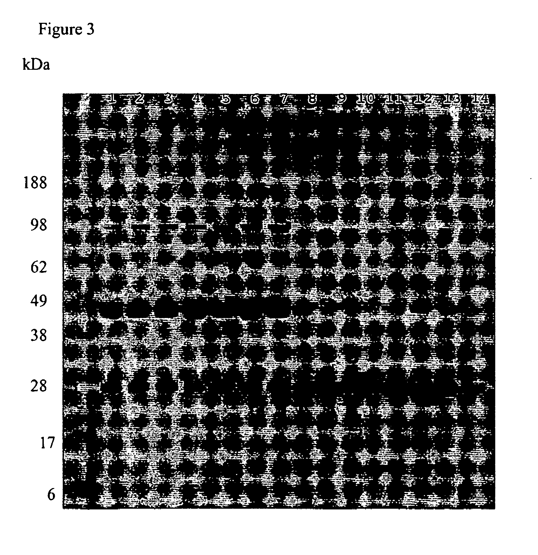

To compare the temperature stability of the Fab' molecules with and without the interchain disulphide during extraction, *E.coli* cells were spiked with purified Fab'. Purified g165 Fab LC-C HC-C, hinge-CAA or g165 Fab LC-S HC-S, hinge-CAA were made up to a final concentration of 2mg/ml Fab' in 100mM Tris.Cl pH7.4 10mM EDTA containing a suspension of plasmid free W3110 *E*. *coli* at 30 OD₆₀₀/ml. 20µl aliquots were incubated in 500µl eppendorf tubes overnight in heating blocks at room temperature, 37°C, 45 °C, 50 °C, 55 °C, 60 °C and 65°C. After the incubation the tubes were centrifuged at 13000g for 1 minute to pellet precipitated proteins and cellular debris, 5µl samples were taken from the supernatant (soluble fraction) and separated using non-reducing SDS-PAGE before staining with Coomassie Blue (Figure 3). 165 Fab' LC-C HC-C hinge-CAA was stable at 65°C (lanes 1-7, major band at approx 50kDa) while 165 Fab' LC-S HC-S hinge-CAA was stable up until 60°C (lanes 8-13, upper band heavy chain dimers, lower band free heavy and light chain), after which protein levels were reduced (lane 14).

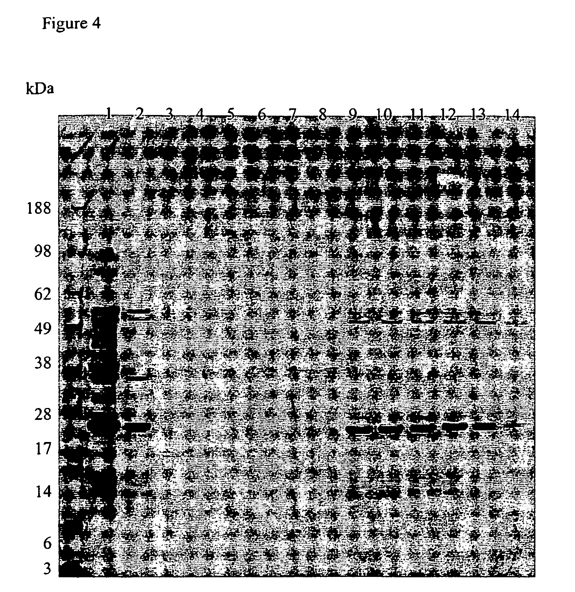

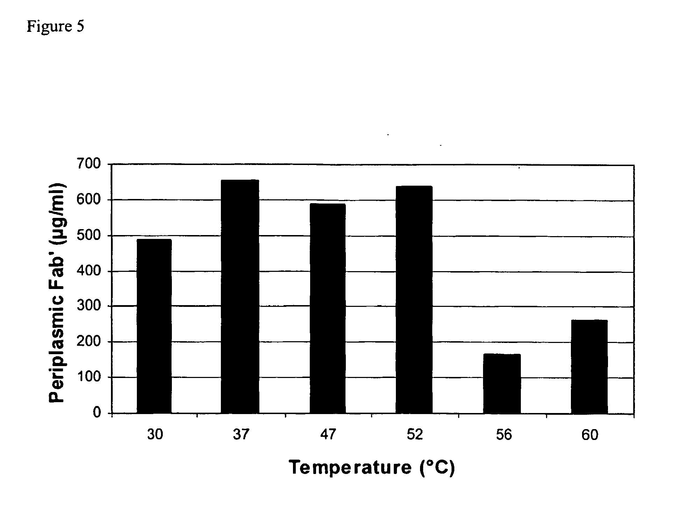

Since SDS-PAGE is denaturing there is no guarantee that the LC, HC and Fab' bands seen on a gel represent correctly folded protein. Hence tests were done to see if Fab' could bind normally to ion exchange and ProteinG column matrices following different temperature incubations. Fermentation cell pastes of 165 Fab' LC-S HC-S hinge-CAA were shaken in Tris / EDTA overnight at 30°C, 37°C, 47°C, 52°C, 56°C and 60°C then tested for Fab' recovery by binding to SP sepharose or Protein G. Figure 4 shows SDS-PAGE of flow through (lanes 3-8) and eluate (lanes 9-14) from SP sepharose and demonstrates that recovery of LC and HC are not affected by temperatures up to and including 56°C (lanes 9-13). At 60°C levels of Fab' recovered from the cation exchange column were reduced (lane 14). Similarly, the Fab' in these Tris / EDTA extracts were found to bind to Protein G even after incubation overnight at temperatures up to 52°C (Figure 5) suggesting that the external surface is correctly folded even after exposure to these elevated temperatures.

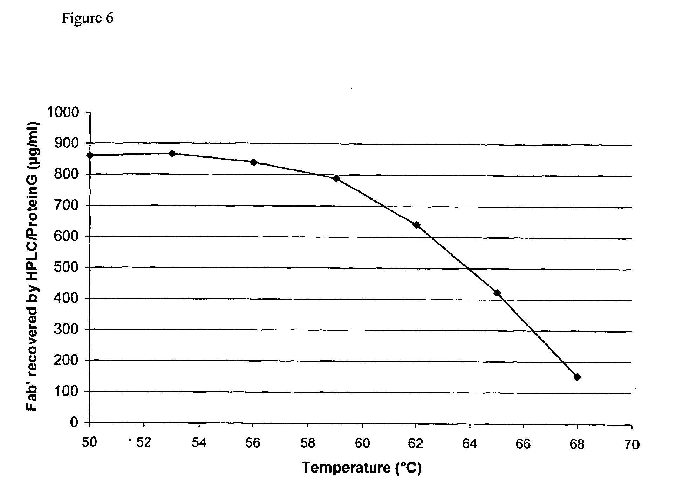

In order to try and pinpoint the critical temperature involved in successful recovery of Fab' from 165 Fab' LC-S HC-S hinge-CAA fermentation cell pastes an overnight extraction at 30°C was performed. A sample was taken out as a reference. Thereafter the extraction was incubated with shaking for one hour at progressively increasing temperatures (50°C, 53°C, 56°C, 59°C, 62°C, 65°C and 68°C). Analysis for binding to protein G in Figure 6 showed that 59°C was the upper cumulative temperature for tolerance at 1 hour for such Fab'.

These data show clearly that the non-covalent interaction between cKappa and Fd is very strong and that surprisingly heat extraction at between 50 and 59°C can still be used to prepare the antibody fragments of the present invention.

### Effect of lack of inter C_{L}:C_{H}1 disulphide bond on the physical performance of Fab' and Fab-PEG.

### i) Purification of Fab'

Fab' engineered to lack inter C_{L}:C_{H}1 disulphide bonds were purified using protein G or ion exchange in exactly the same manner as Fab' containing inter C_{L}:C_{H}1 disulphide bonds. Since these involved elution at pH 2.7 (protein G) or equilibration at pH 4.5 (ion exchange) the Fab' interaction between the HC and LC was clearly stable.

### ii) Antigen binding affinity in vitro.

g8156 Fab' with one or two PEG molecules attached to the hinge in the presence or absence of a covalent linkage between LC and HC were analysed for antigen affinity using surface plasmon resonance. Table 2 shows that neither the lack of inter C_{L}:C_{H}1 disulphide or presence of di-PEGylation materially affects the binding affinity.

**Table 2. Antigen affinity of mono and di-PEGylated Fab' in vitro.**

| **Fab'** | **PEG** | **KD (nM)** |
|---|---|---|
| g8516 LC-C HC-C, hinge-CAA | 1 x 40kDa | 0.10 |
| g8516 LC-S HC-S, hinge-CAA | 1 x 40kDa | 0.13 |
| g8516 LC-C HC-C, hinge-CPPCPA | 2 x 20kDa | 0.10 |
| g8516 LC-S HC-S, hinge-CPPCPA | 2 x 20kDa | 0.31 |

### Example 3 Pharmacokinetics of Fab'-PEG in rats.

### i) Circulating half life of Fab' PEGylated on the hinge

PEGylation in the hinge of Fab' followed by ¹²⁵I labelling and intravenous or sub cutaneous injection into rats enables analysis of the serum permanence of potential therapeutic Fab'. The circulating half life of non-PEGylated Fab' is very short (t½β ≈ 30 minutes) and that of free LC or HC is likely to be shorter still. ¹²⁵I labelling of proteins is random. Hence Fab' that are PEGylated only on the HC and lack inter C_{L}:C_{H}1 disulphide bonds will lose approximately half of the injected ¹²⁵I blood borne radioactivity within a few hours if LC dissociates from HC *in vivo.*

300µg of Fab'-PEG per animal group was ¹²⁵I-labelled using Bolton and Hunter reagent (Amersham) to a specific activity of 0.22 - 0.33 µCi/µg. Male Sprague Dawley rats of 220-250 g (Harlan) were injected *intravenously* or *subcutaneously* with 20 µg ¹²⁵I-labelled Fab'-PEG variants whilst under Halothane anaesthesia (n = 6 per group). Serial arterial bleeds from the tail were taken at 0.5, 2, 4, 6, 24, 48, 72 and 144 hours post administration. Samples were counted using a COBRA^{™} Autogamma counter (Canberra Packard). Data were plotted and Area Under Curve were calculated using GraphPad Prism (GraphPad Software Incorporated) and is expressed as % injected dose.hour (% i.d/hr). The t½α is defined by time points 0.5, 2, 4, and 6, whilst the t½β is defined by time points 24, 48, 72 and 144. The means and standard errors of means (SEM) of data for all six animals are shown in Table 3.

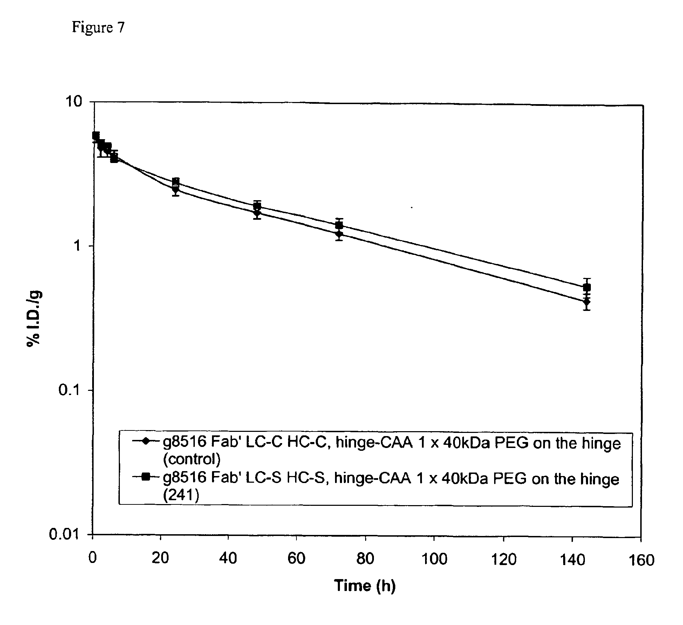

g8516 Fab' LC-S HC-S hinge-CAA and g8516 Fab' LC-C HC-C hinge-CAA were modified with a single hinge branched 40kDa PEG using TCEP as the reductant and subjected to such a pharmacokinetic analysis in rats.
In an experiment where the Fab' were injected *intravenously* the half lives and curve shapes of these two materials were essentially identical (Table 3 and Figure 7) suggesting that the LC and HC remain partnered even up to 6 days after injection into the rat circulation.

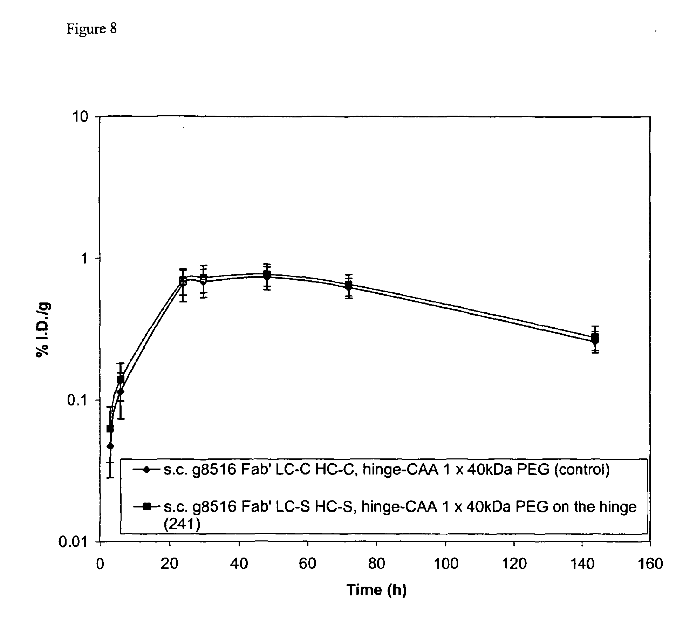

Again, in an experiment where the Fab' were injected *subcutaneously* and the radioactivity sampled from the blood, the lack of covalent interaction between LC and HC did not affect the circulating half life (Table 3 and Figure 8).

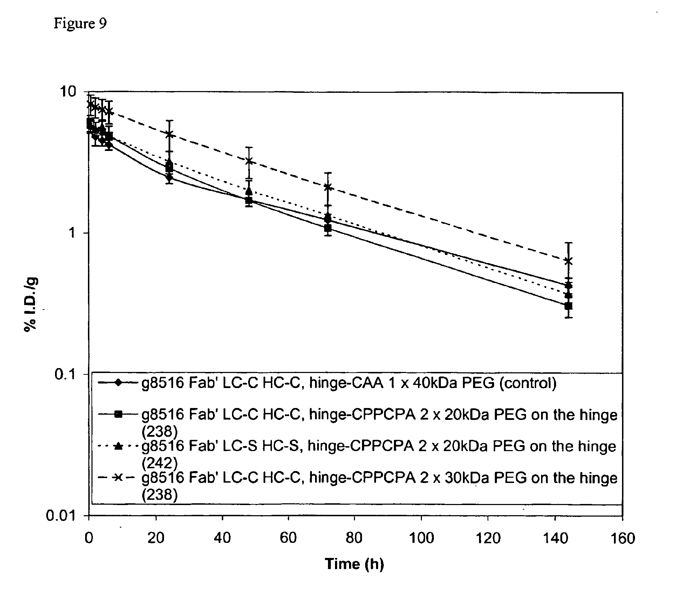

When the Fab' hinge is extended to include the middle hinge of a human γ1 isotype antibody there are two cysteines available for attachment of PEG. The data for when the hinge was di-PEGylated with 2 x 20kDa linear PEG (Table 3 and Figure 9) show that the pharmacokinetics are very similar to that of the mono-PEGylated Fab' control. There is a slight difference in the curve shape for the di-PEGylated Fab' as opposed to the mono-PEGylated Fab' and hence as a result it is not possible to calculate an α phase half life for the di-PEGylated material. The half life of the di-PEG-Fab' is the same irrespective of whether the LC and HC are covalently associated or not.

**Table 3. Pharmacokinetic analysis of Fab-PEG in rat model.**

| **Fab** | **PEG** | **Admin.** | **t ½ α (h)** | **T½ β (h)** | **AUC (0-∞) (%dose*h)** |
|---|---|---|---|---|---|
| g8516 Fab' LC-C HC-C Hinge-CAA | 1x40kDa (branched) | *i.v*. | 4.76 ± 1.3 | 48 ± 2.8 | 4554 ± 268 |
| g8516 Fab' LC-S HC-S Hinge-CAA | 1x40kDa (branched) | *i.v*. | 4.2 ± 1.0 | 51 ± 2.8 | 5307 ± 600 |
| g8516 Fab' LC-C HC-C Hinge-CPPCPA | 2x20kDa | *i.v*. | - | 36 ± 1.9 | 4671 ± 538 |
| g8516 Fab' LC-C HC-C hinge-CPPCPA | 2x30kDa | *i.v*. | - | 39 ± 3.6 | 6734 ± 1693 |
| g8516 Fab' LC-S HC-S Hinge-CPPCPA | 2x20kDa | *i.v*. | - | 37 ± 3.9 | 5224 ± 748 |
| | | | | | |
| | | | | | |
| g8516 Fab' LC-C HC-C Hinge-CAA | 1x40kDa (branched) | *s.c*. | - | 47 ± 6.5 | 2061 ± 375 |
| g8516 Fab' LC-S HC-S Hinge-CAA | 1x40kDa (branched) | *S.C*. | - | 50 ± 1.9 | 2014 ± 313 |

### Example 4 Mouse antigen binding efficacy models: In vivo efficacy in animal models.

Further evidence of the resilience of the interaction between LC and HC is found in the ability of Fab' lacking inter C_{L}:C_{H}1 disulphide bonds to neutralise antigen in mouse models. The human cytokine, IL-1β, antigen for g85·16 Fab' elicits biological responses from mice when injected *i.v.* (IL-6 production) or i.p. (neutrophil migration) and these can be blocked or inhibited by neutralising g8516 Fab-PEG. Antibodies have 6 CDR's (3 on each polypeptide) that are normally involved in antigen binding. Hence administration of monoPEGylated forms of g8516 Fab' LC-S HC-S hinge-CAA and g8516 Fab' LC-C HC-C hinge-CAA may be expected to block these antigen driven events if the LC and HC remain associated together. Unpairing and subsequent loss from the circulation of the non-PEGylated LC would be expected to result in loss of neutralisation ability.

### i) i.v. dosed g8516 Fab'-PEG and i.v. dosed hIL-1β.

At time t=-15 min; Male Balb/c mice (19.9g) mice injected intravenously (*i.v.*) with test mAb at 1, 3 and 10mg/kg in 100µl PBS, or PBS vehicle alone (under halothane anaesthesia). At t= 0; mice injected i.v. with antigen (30µg/kg) in 100µl PBS vehicle, or PBS (alone under halothane anaesthesia). At t=90 min; cardiac puncture was performed and blood drawn into heparinised saline (100 U/ml heparin) (under halothane anaesthesia). Mice were then killed by cervical dislocation.

Plasma was prepared from the sampled blood by centrifugation (14000 x g, 2 min, RT) and stored at -20°C prior to determination of IL-6 levels by ELISA. IL-6 ELISA was performed according to manufacturers instructions (BD Pharmingen OPT-EIA mIL-6). Samples were diluted 1/16.

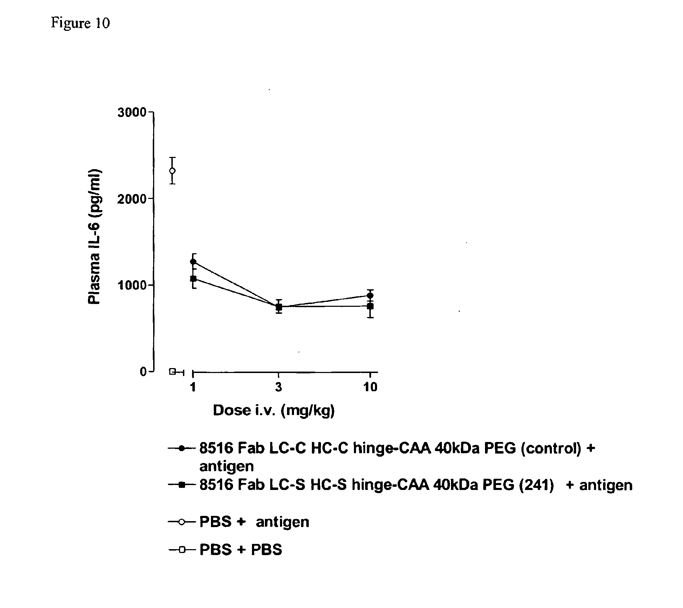

Figure 10 shows that g8516 Fab' LC-S HC-S hinge-CAA was equally efficacious as g8516 Fab' LC-C HC-C hinge-CAA in neutralising antigen when administered i.v. at all doses tested.

### ii) i.v dosed g8516 Fab'-PEG and intraperitoneal dosed hIL-1β.

The i.v. pre-dosing of Fab-PEG was extended to 1, 3 and 7 days prior to i.p. administration of the antigen in a different efficacy model.

Male Balb/c mice (21 g) were injected intravenously (i. v.) with a single dose (3 mg/kg in 100 µl PBS) of g8516 Fab'LC-C HC-C hinge-CAA-40kDa PEG, g8516 Fab'LC-S HC-S hinge-CAA-40kDa PEG, or ghA33 Fab'LC-C HC-C hinge-CAA-40kDa PEG (irrelevant control), 1, 3 or 7 days prior to an i.p. injection of hIL-1β (3 ng/kg in 100 µl PBS vehicle). After 120 minutes, mice were killed by cervical dislocation and peritoneal lavage was performed (3ml PBS + 0.25% BSA, 12mM HEPES). A total leukocyte count was performed using a Coulter Counter. For identification ofneutrophils, 50 µl peritoneal lavage fluid was stained with 1:300 dilution of anti-CD45-CyChrome mAb and 1:300 dilution of anti-GR-1-PE mAb (anti-Ly6G/Ly6C) for 20 minutes (4°C, in the dark). Leukocytes were washed once in PBS (0.25% BSA, 12mM HEPES), resuspended in 300µl PBS (0.25% BSA, 12mM HEPES) and analysed by flow cytometry. Neutrophils were identified as CD45⁺GR-1^{HIGH}.

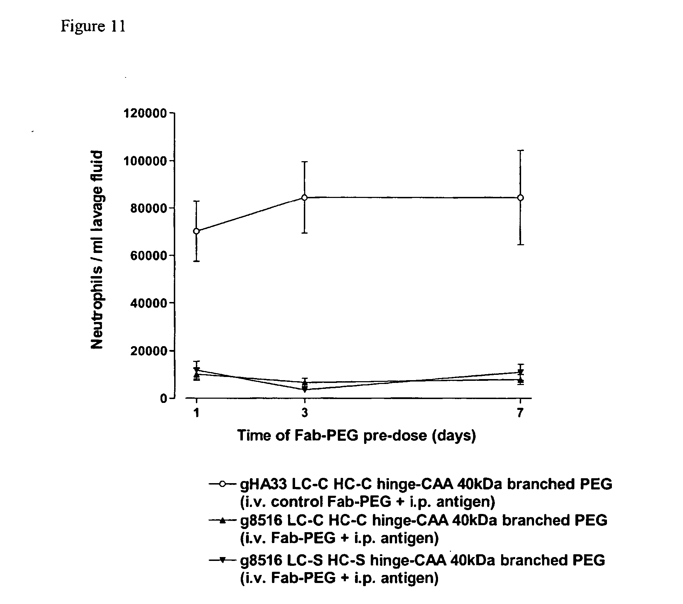

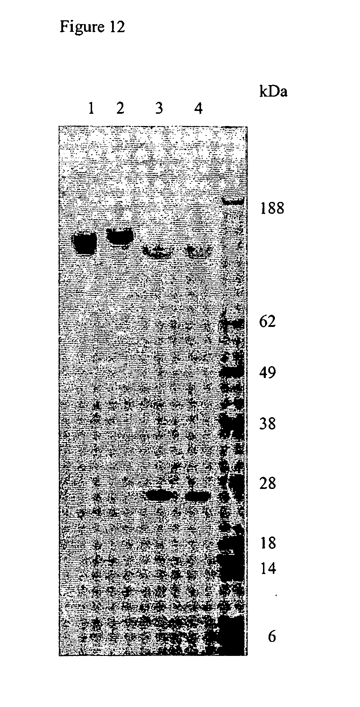

Figure 11 shows that there was no difference between g8516 Fab-PEG that have, or lack inter C_{L}:C_{H}1 disulphide bonds at any of the time points. This demonstrates that efficacy is retained during 1 week in the mouse circulation and therefore by implication that LC and HC remain associated during this time. Non reducing SDS-PAGE of g8516 Fab-PEG material used in these animal models is shown in Figure 12. This figure demonstrates that the LC and HC association is non-covalent for g8516 Fab' LC-S HC-S, hinge-CAA-PEG (lanes 3 and 4, higher molecular weight band is heavy chain with PEG attached to the hinge, lower molecular weight band is free light chain) compared to the covalent linkage observed in g8516 Fab' LC-C HC-C, hinge CAA (lanes 1 and 2 single high molecular weight band demonstrating covalent linkage).

From the above examples it can clearly be seen that the novel PEGylated molecules of the present invention can be produced more efficiently than PEGylated antibodies that contain an inter C_{L}:C_{H}1 disulphide bond. The examples also demonstrate that PEGylation of Fab' which lack the interchain disulphide bond has no adverse effects on the biological activity or stability of the antibody Fab' making these useful therapeutic molecules which can be produced more efficiently than conventional Fab'.

## Claims

1. An antibody Fab' fragment to which at least one effector molecule is attached **characterized in that** the hinge contains one or two cysteines and the heavy chain in the fragment is not covalently bonded to the light chain and both the interchain cysteine of C_{L} and the interchain cysteine of C_{H}1 have been replaced with another amino acid and wherein each effector molecule is PEG and each effector molecule is attached to a cysteine in the hinge region.

2. The antibody fragment of claim 1 wherein the interchain cysteine of C_{L} and the interchain cysteine of C_{H}1 have been replaced with a non-thiol containing amino acid.

3. The antibody fragment of claim 2 wherein the interchain cysteine of C_{L} has been replaced with serine.

4. The antibody fragment of claim 2 wherein the interchain cysteine of C_{H}1 has been replaced with serine.

5. The antibody fragment of claim 2 wherein both the interchain cysteine of C_{H}1 and the interchain cysteine of C_{L} have been replaced with serine.

6. The antibody fragment of claims 1-5 wherein the interchain cysteine of C_{L} is at position 214 of the light chain and the interchain cysteine of C_{H}1 is at position 233 of the heavy chain.

7. An antibody Fab' fragment according to claims 1-6 that contains a modified hinge region that differs in length and/or composition from the native hinge region.

8. An antibody Fab' fragment according to claim 7 in which the modified hinge contains 1 cysteine residue.

9. An antibody Fab' fragment according to claim 8 in which the hinge comprises the sequence in SEQ ID NO: 1 or SEQ ID NO:2.

10. The antibody fragment of claim 7 in which the modified hinge contains 2 cysteine residues.

11. An antibody Fab' fragment according to claim 10 in which the hinge comprises the sequence in SEQ ID NO:3 or SEQ ID NO:4.

12. An antibody Fab' fragment according to claim 1 in which two effector molecules are attached to the hinge region.

13. A method of producing an antibody Fab' fragment according to claims 1-12 comprising:
a. treating an antibody Fab' fragment in which both the interchain cysteine of C_{L} and the interchain cysteine of C_{H}1 have been replaced with another amino acid and the hinge contains one or two cysteines with a trialkylphosphine reductant
b. reacting the treated fragment with an effector molecule wherein the effector molecule is PEG.

14. The method according to claim 13 in which the trialkylphosphine reductant is tris(2-carboxyethyl)phosphine (TCEP).

15. The method according to claim 14 in which the trialkylphosphine reductant is tris(3-hydroxypropyl)phosphine (THP).

16. The method according to claim 13 in which either or both of steps (a) and (b) are performed in the presence of EDTA.

17. The method according to claim 16 in which both steps (a) and (b) are performed in the presence of EDTA.

18. A pharmaceutical composition comprising an antibody fragment according to any of the preceding claims, together with one or more pharmaceutically acceptable excipients, diluents or carriers.

## Patentansprüche

1. Fab`-Antikörperfragment, an das wenigstens ein Effektormolekül gebunden ist, **dadurch gekennzeichnet, dass** das Scharnier ein oder zwei Cysteine enthält und die schwere Kette in dem Fragment nicht kovalent an die leichte Kette gebunden ist und sowohl das Brückencystein von C_{L} als auch das Brückencystein von C_{H}1 durch eine andere Aminosäure ersetzt wurden, und wobei es sich bei den Effektormolekülen jeweils um PEG handelt und die Effektormoleküle jeweils an ein Cystein im Scharnierbereich gebunden sind.

2. Antikörperfragment nach Anspruch 1, wobei das Brückencystein von C_{L} und das Brückencystein von C_{H}1 durch eine nicht thiolhaltige Aminosäure ersetzt wurden.

3. Antikörperfragment nach Anspruch 2, wobei das Brückencystein von C_{L} durch Serin ersetzt wurde.

4. Antikörperfragment nach Anspruch 2, wobei das Brückencystein von C_{H}1 durch Serin ersetzt wurde.

5. Antikörperfragment nach Anspruch 2, wobei sowohl das Brückencystein von C_{H}1 als auch das Brückencystein von C_{L} durch Serin ersetzt wurden.

6. Antikörperfragment nach Anspruch 1-5, wobei das Brückencystein von C_{L} in Position 214 der leichten Kette und das Brückencystein von C_{H}1 in Position 233 der schweren Kette liegt.

7. Fab'-Antikörperfragment gemäß Anspruch 1-6, das einen modifizierten Scharnierbereich enthält, der sich in Länge und/oder Zusammensetzung vom nativen Scharnierbereich unterscheidet.

8. Fab'-Antikörperfragment gemäß Anspruch 7, bei dem das modifizierte Scharnier 1 Cysteinrest enthält.

9. Fab'-Antikörperfragment gemäß Anspruch 8, bei dem das Scharnier die Sequenz in SEQ ID NO:1 oder SEQ ID NO:2 umfasst.

10. Antikörperfragment nach Anspruch 7, bei dem das modifizierte Scharnier 2 Cysteinreste enthält.

11. Fab'-Antikörperfragment gemäß Anspruch 10, bei dem das Scharnier die Sequenz in SEQ ID NO:3 oder SEQ ID NO:4 umfasst.

12. Fab'-Antikörperfragment gemäß Anspruch 1, bei dem zwei Effektormoleküle an den Scharnierbereich gebunden sind.

13. herfahren zur Herstellung eines Fab'-Antikörperfragments gemäß Anspruch 1-12, wobei man:
a. ein Fab'-Antikörperfragment, bei dem sowohl das Brückencystein von C_{L} als auch das Brückencystein von C_{H}1 durch eine andere Aminosäure ersetzt wurden und das Scharnier ein oder zwei Cysteine enthält, mit einem Trialkylphosphin-Reduktionsmittel versetzt,
b. das behandelte Fragment mit einem Effektormolekül umsetzt, wobei es sich bei dem Effektormolekül um PEG handelt.

14. Verfahren gemäß Anspruch 13, bei dem es sich bei dem Trialkylphosphin-Reduktionsmittel um Tris(2-carboxyethyl)phosphin (TCEP) handelt.

15. herfahren gemäß Anspruch 14, bei dem es sich bei dem Trialkylphosphin-Reduktionsmittel um Tris(3-hydroxypropyl)phosphin (THP) handelt.

16. Verfahren gemäß Anspruch 13, bei dem einer der Schritte (a) und (b) oder beide Schritte in Gegenwart von EDTA ausgeführt wird bzw. werden.

17. Verfahren gemäß Anspruch 16, bei dem sowohl Schritt (a) als auch Schritt (b) in Gegenwart von EDTA ausgeführt werden.

18. Pharmazeutische Zusammensetzung, umfassend ein Antikörperfragment gemäß einem der vorhergehenden Ansprüche zusammen mit einem oder mehreren pharmazeutisch unbedenklichen Hilfs-, Verdünnungs- oder Trägermitteln.

## Revendications

1. Fragment Fab' d'anticorps auquel est attachée au moins une molécule effectrice, **caractérisé en ce que** la charnière contient une ou deux cystéine(s), que la chaîne lourde dans le fragment n'est pas liée, de manière covalente, à la chaîne légère et qu'à la fois la cystéine de l'inter-chaîne du C_{L} et la cystéine de l'inter-chaîne du C_{H}1 ont été remplacées par un autre acide aminé, dans lequel chaque molécule effectrice est le PEG et chaque molécule effectrice est attachée à une cystéine dans la région charnière.

2. Fragment d'anticorps selon la revendication 1, dans lequel la cystéine de l'inter-chaîne du C_{L} et la cystéine de l'inter-chaîne du C_{H}1 ont été remplacées par un acide aminé ne contenant pas de thiol.

3. Fragment d'anticorps selon la revendication 2, dans lequel la cystéine de l'inter-chaîne du C_{L} a été remplacée par une sérine.

4. Fragment d'anticorps selon la revendication 2, dans lequel la cystéine de l'inter-chaîne du C_{H}1 a été remplacée par une sérine.

5. Fragment d'anticorps selon la revendication 2, dans lequel à la fois la cystéine de l'inter-chaîne du C_{H}1 et la cystéine de l'inter-chaîne du C_{L} ont été remplacées par une sérine.

6. Fragment d'anticorps selon les revendications 1 à 5, dans lequel la cystéine de l'inter-chaîne du C_{L} est à la position 214 de la chaîne légère et la cystéine de l'inter-chaîne du C_{H}1 est à la position 233 de la chaîne lourde.

7. Fragment Fab' d'anticorps, selon les revendications 1 à 6, contenant une région charnière modifiée, qui diffère de la région charnière naturelle de par la longueur et/ou la composition.

8. Fragment Fab' d'anticorps selon la revendication 7, dans lequel la charnière modifiée contient 1 résidu de cystéine.

9. Fragment Fab' d'anticorps selon la revendication 8, dans lequel la charnière comprend la séquence dans la SEQ ID n° : 1 ou la SEQ ID n° : 2.

10. Fragment d'anticorps selon la revendication 7, dans lequel la charnière modifiée contient 2 résidus de cystéine.

11. Fragment Fab' d'anticorps selon la revendication 10, dans lequel la charnière comprend la séquence dans la SEQ ID n° : 3 ou la SEQ ID n° : 4.

12. Fragment Fab' d'anticorps selon la revendication 1, dans lequel deux molécules effectrices sont attachées à la région charnière.

13. Procédé de production d'un fragment Fab' d'anticorps, selon les revendications 1 à 12, comprenant les étapes consistant à :
a. traiter un fragment Fab' d'anticorps, dans lequel à la fois la cystéine de l'inter-chaîne du C_{L} et la cystéine de l'inter-chaîne du C_{H}1 ont été remplacées par un autre acide aminé et la charnière contient une ou deux cystéine(s) avec un réducteur à trialkylphosphine
b. faire réagir le fragment traité avec une molécule effectrice, dans lequel la molécule effectrice est le PEG.

14. Procédé selon la revendication 13, dans lequel le réducteur à trialkylphosphine est la tris(2-carboxyéthyl)phosphine (TCEP).

15. Procédé selon la revendication 14, dans lequel le réducteur à trialkylphosphine est la tris(3-hydroxypropyl)phosphine (THF).

16. Procédé selon la revendication 13, dans lequel soit l'étape (a), soit l'étape (b), soit les deux sont effectuées en présence d'EDTA.

17. Procédé selon la revendication 16, dans lequel les étapes (a) et (b) sont toutes deux effectuées en présence d'EDTA.

18. Composition pharmaceutique comprenant un fragment d'anticorps, selon l'une quelconque des revendications précédentes, conjointement avec un ou plusieurs excipient(s), diluant(s) ou transporteur(s) acceptable(s) d'un point de vue pharmaceutique.
